(19) Europäisches Patentamt
European Patent Office
Office européen des brevets

(11) EP 1 413 888 A1

(12) EUROPEAN PATENT APPLICATION
published in accordance with Art. 158(3) EPC

(43) Date of publication:
28.04.2004 Bulletin 2004/18

(51) Int Cl.⁷: G01N 35/00, G01N 33/48

(21) Application number: 02751815.8

(86) International application number:
PCT/JP2002/007758

(22) Date of filing: 30.07.2002

(87) International publication number:
WO 2003/012452 (13.02.2003 Gazette 2003/07)

(84) Designated Contracting States:
AT BE BG CH CY CZ DE DK EE ES FI FR GB GR
IE IT LI LU MC NL PT SE SK TR

(30) Priority: 31.07.2001 JP 2001232604
26.02.2002 JP 2002050145

(71) Applicants:
• A & T Corporation
Fujisawa-shi, Kanagawa 252-0816 (JP)
• Japan as respresented by the president of Kochi
Medical School
Nankoku-shi, Kochi 783-8505 (JP)

(72) Inventors:
• HORIMOTO, Akira, c/o A & T CORPORATION
Fujisawa-shi, Kanagawa 252-0816 (JP)
• KATAOKA, Hiromi
Kochi-shi, Kochi 781-5108 (JP)

(74) Representative: Muschke, Markus, Dipl.-Phys.
Patentanwälte Dipl.-Ing. Schwabe, Dr.Dr.
Sandmair, Dr. Marx,
Stuntzstrasse 16
81677 München (DE)

(54) **CLINICAL EXAMINATION ANALYZING DEVICE, CLINICAL EXAMINATION ANALYZING METHOD AND CLINICAL EXAMINATION ANALYZING PROGRAM**

(57)    The clinical test analyzing device (100) includes an appearance pattern storing section (301) that stores a plurality of appearance patterns on analyzing results of a prescribed plurality of items in clinical tests for a prescribed population, a clinical test result input section (302) that inputs analyzing results of the prescribed plurality of items in clinical tests for a subject, and a resemblance calculation section (303) that calculates the degree of resemblance of the analyzing result to the most resembling appearance pattern, based on the analyzing results that was input by the clinical test result input section (302) and the plurality of appearance patterns stored in the appearance pattern storing section (301).

FIG.3

EP 1 413 888 A1

**Description**

TECHNICAL FIELD

The present invention relates to a clinical test analyzing device, a clinical test analyzing method, and a computer program for analyzing a plurality of analyzing items with a plurality of appearance patterns.

BACKGROUND ART

Conventionally, in a clinical test, each sample (specimen) provided by a person being tested (hereinafter "a subject") is analyzed for prescribed items, and the subject's condition is judged based on the results of the analysis. Particularly in these days, there has been a great advancement in automatic analyzing devices. Such automatic analyzing devices are installed at the place where the clinical test is necessary, i.e., test rooms of hospitals, and can analyze a number of samples in a short time. Owing to the advancement of clinical test technology, it is now possible to perform a test of high reliability and judge the subject's condition based on the test results.
However, the conventional technology has problems as follows:
Since judgment of the analyzing results of the clinical test is standardized, even more samples may be disadvantageously detected as the ones requiring a re-test or a further precise test than they should be. Such a problem is described below in detail.
Firstly, a case, where the above problem is hard to occur, is described. Presently, majority of companies let the employees take a physical checkup periodically. Since most employees are in good health, few employees have abnormal values in some tests. For instance, few employees have abnormal values in blood tests. Therefore, it is possible to distinguish ones who need a re-test or a further precise test from healthy persons by setting a standardized criterion for judgment. In other words, in the case of a clinical test for a population of healthy persons, it is enough to set a normal value range for each test item and take a person with even one item out of the range as the one who needs a re-test or a further precise test.
Secondly, let us consider patients in hospital as a case where the above problem is apt to occur. Inpatients are subject to take clinical tests periodically in order to monitor the improvement by medical treatments. Since inpatients are with certain kind of disease, if the above standardized criterion for judgment is applied, most are to be classified as the ones who need a re-test or a further precise test. Since this ruins the original purpose of medical treatment and also degrades reliability of the judgment to loose up on the standardized criterion to widely set the normal value range, it is not possible to take such a selection. Namely, in the case of a clinical test for a population with disease, a problem occurs that the judgment is not possible whether the abnormal value that normally comes out is the very one requiring a re-test or a further precise test, or the one that can be accepted as normal.
On the other hand, there is another method of previous value check where the judgment, whether the abnormal values are acceptable, is made by comparing the previous test results with the present test results for the same patient. However, since most patients show abnormal values for a plurality of items, a problem is that it is quite difficult to judge without being a skilled doctor. Besides, even with the previous value check method, since a similar standardized judgment is made whether the value fits in a fixed range in multi-dimensional space having an axis for each item, the problem that a number of samples that need a re-test or a further precise test are detected still exists.
It is an object of the present invention to rationally eliminate unnecessary re-tests while maintaining reliability of the test.

DISCLOSURE OF THE INVENTION

The clinical test analyzing device according to the present invention includes an appearance pattern storing section that stores a plurality of appearance patterns on past analyzing results of a plurality of prescribed items in a clinical test for a prescribed population, a clinical test result input section that acquires current analyzing results of the plurality of prescribed items in the clinical test for a subject, and a resemblance calculation section that calculates the degree of resemblance of the current analyzing results to the most resembling appearance pattern, by comparing the current analyzing results with the appearance patterns.
Thereby, the present invention makes it possible to judge whether a re-test is definitely necessary or not, based on the appearance patterns.
The clinical test analyzing device according to the present invention is characterized by the fact that it takes a population of patients as the prescribed population.
Namely, the present invention makes it possible to judge whether an abnormal value is an acceptable pattern or a test result that requires a definite re-test, based on the abnormal value pattern.
The clinical test analyzing device according to the present invention is characterized by the fact that the plurality of appearance patterns is extracted from the prescribed population.

Namely, the present invention makes it possible to obtain a pattern of phenomenologically strong correlation on the analyzing result of the plurality of items, and judge whether it is an acceptable pattern or a test result that requires a definite re-test. Furthermore, when calculating appearance patterns, it is possible to use the data mining method, such as the machine learning, the neural network, and the self organization map.

The clinical test analyzing device according to the present invention is characterized by the fact that the clinical test includes either at least one test or a combination of more than one test among microscopic examination of urinary sediment, blood morphology/function test, hemorrhage/blood coagulation test, blood chemical test, endocrinological test, tumor marker, infection serologic test, self-antibody test, and immunological test for plasma protein.

Namely, the present invention makes it possible to judge whether an abnormal value is an acceptable pattern or a test result that requires a definite re-test, based on the appearance patterns corresponding to the classification of clinical tests according to the National Health Insurance (NHI) point.

The clinical test analyzing device according to the present invention is characterized by the fact that the prescribed plurality of items are values of numerical quantity or concentration of white blood corpuscle, red blood corpuscle, hemoglobin, hematocrit, and blood platelet.

Namely, the present invention makes it possible to judge whether an abnormal value is an acceptable pattern or a test result that requires a definite re-test, using the appearance patterns based on the blood test that is most frequently conducted in the medical field in practice.

The clinical test analyzing device according to the present invention is characterized by the fact that the prescribed plurality of items are values of concentration of sodium (Na), potassium (K), and chloride (Cl) in an electrolyte.

Namely, the present invention makes it possible to judge whether an abnormal value is an acceptable pattern or a test result that requires a definite re-test, using the appearance patterns based on the electrolyte test that is most frequently conducted in the medical field in practice.

The clinical test analyzing device according to the present invention is characterized by the fact that the analyzing results of the prescribed plurality of items are the analyzing results that are compared with those of the previous test in item by item for every single member of the prescribed population.

Namely, the present invention makes it possible to judge whether an abnormal value is an acceptable pattern or a test result that requires a definite re-test, based on the appearance patterns that takes the previous clinical test results and time variation of the test value into consideration.

The clinical test analyzing device according to the present invention is characterized by the fact that it is further equipped with a re-test judgment section that judges whether the subject needs a re-test or not, based on the degree of resemblance calculated by the resemblance calculation section and a preset judgment threshold.

The present invention makes a judgment whether an abnormal value is an acceptable pattern or a test result that requires a definite re-test, when the test result shows that the degree of resemblance to any appearance pattern is small (i.e., a chance of being abnormal is quite high).

The clinical test analyzing method according to the present invention includes acquiring current analyzing results of a plurality of prescribed items in a clinical test for a subject, acquiring a plurality of appearance patterns on past analyzing results of the prescribed items in the clinical test for a prescribed population, and detecting whether each of the current analyzing results is an abnormal result of a slim chance of occurrence, by comparing the current analyzing results with the appearance patterns.

Namely, the present invention makes it possible to judge whether a re-test is definitely necessary or not, based on the appearance patterns.

The clinical test analyzing method according to the present invention is characterized by the fact that it takes a population of patients as the prescribed population.

Namely, the present invention makes it possible to judge whether an abnormal value is an acceptable pattern or a test result that requires a definite re-test, based on the abnormal value pattern.

The clinical test analyzing method according to the present invention is characterized by the fact that the plurality of appearance patterns is extracted from the prescribed population. Namely, the present invention makes it possible to obtain a pattern of phenomenologically strong correlation on the analyzing result of the plurality of items, and judge whether it is an acceptable pattern or a test result that requires a definite re-test. Furthermore, when calculating appearance patterns, it is possible to use the data mining method, such as the machine learning, the neural network, and the self organization map.

The clinical test analyzing method according to the present invention is characterized by the fact that the analyzing results of the prescribed plurality of items are the analyzing results that are compared with those of the previous test in item by item for every single member of the prescribed population.

Namely, the present invention makes it possible to judge whether an abnormal value is an acceptable pattern or a test result that requires a definite re-test, based on the appearance patterns that takes the previous clinical test results and time variation of the test value into consideration.

The computer program according to the present invention makes a computer realize acquiring current analyzing results of a plurality of prescribed items in a clinical test for a subject, acquiring a plurality of appearance patterns on past analyzing results of the prescribed plurality of items in a clinical test for a prescribed population, and detecting whether each of the current analyzing results is an abnormal result of a slim chance of occurrence, by comparing the current analyzing results with the appearance patterns.

Namely, the present invention makes it possible to judge whether a re-test is definitely necessary or not, based on the appearance patterns.

The computer program according to the present invention is characterized by the fact that it takes a population of patients as the prescribed population.

Namely, the present invention makes it possible to judge whether an abnormal value is an acceptable pattern or a test result that requires a definite re-test, based on the abnormal value pattern.

The computer program according to the present invention is characterized by the fact that the plurality of appearance patterns is extracted from the prescribed population.

Namely, the present invention makes it possible to obtain a pattern of phenomenologically strong correlation on the analyzing result of the plurality of items, and judge whether it is an acceptable pattern or a test result that requires a definite re-test. Furthermore, when calculating appearance patterns, it is possible to use the data mining method, such as the machine learning, the neural network, and the self organization map.

The computer program according to the present invention is characterized by the fact that the analyzing results of the prescribed plurality of items are the analyzing results that are compared with those of the previous test in item by item for every single member of the prescribed population.

Namely, the present invention makes it possible to judge whether an abnormal value is an acceptable pattern or a test result that requires a definite re-test, based on the appearance patterns that takes the previous clinical test results and time variation of the test value into consideration.

BRIEF DESCRIPTION OF THE DRAWINGS

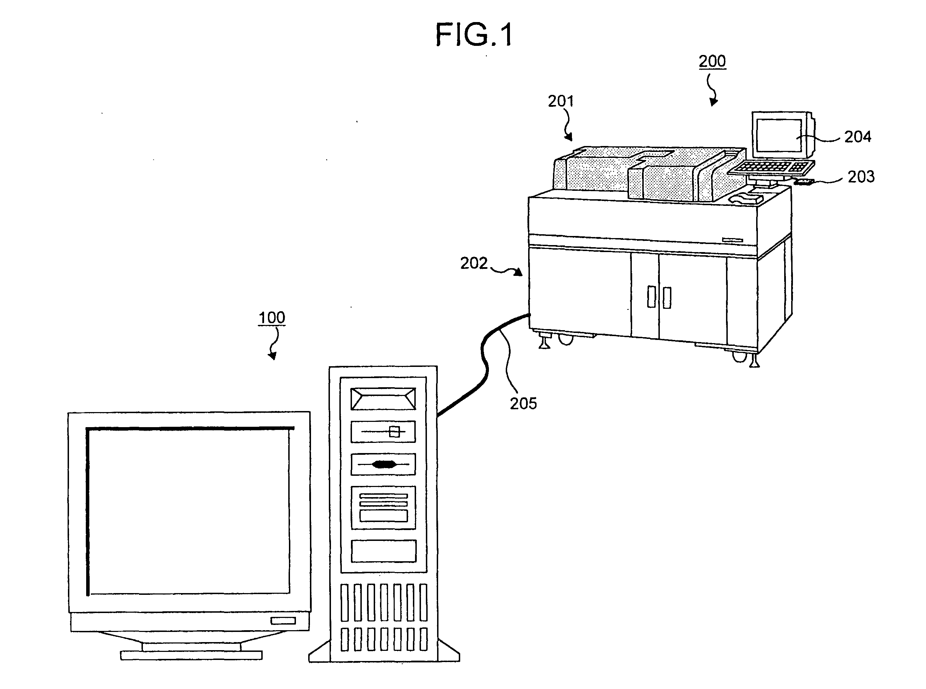

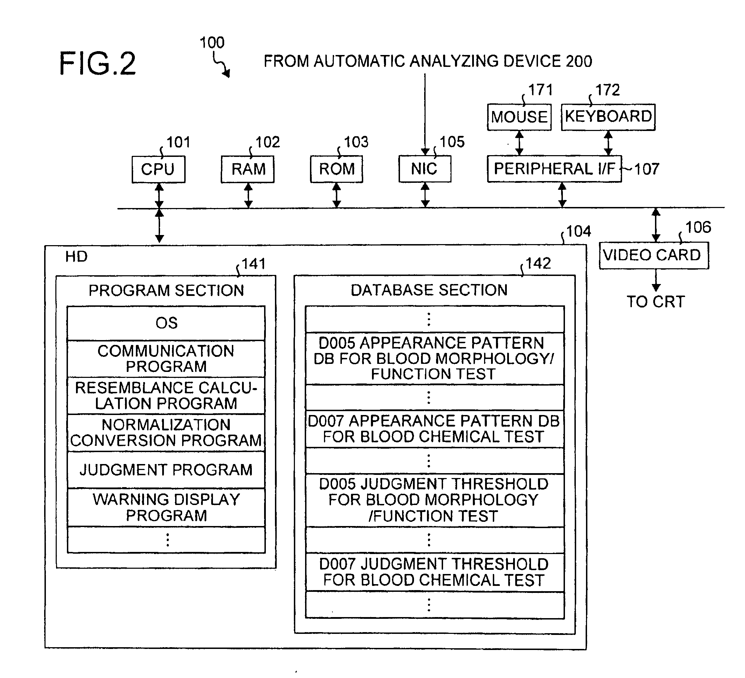

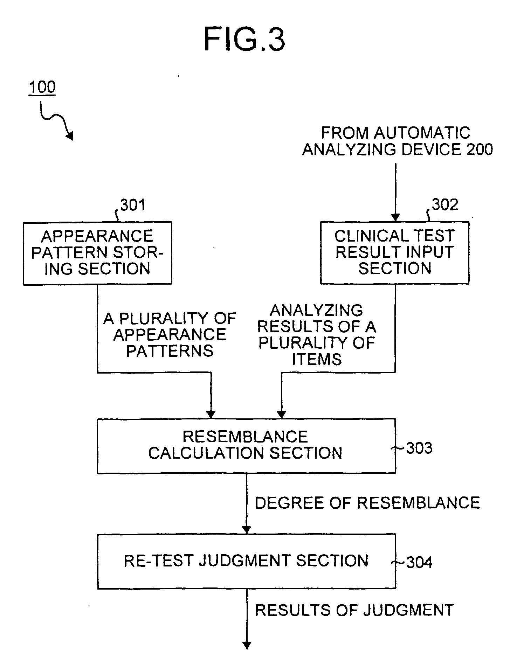

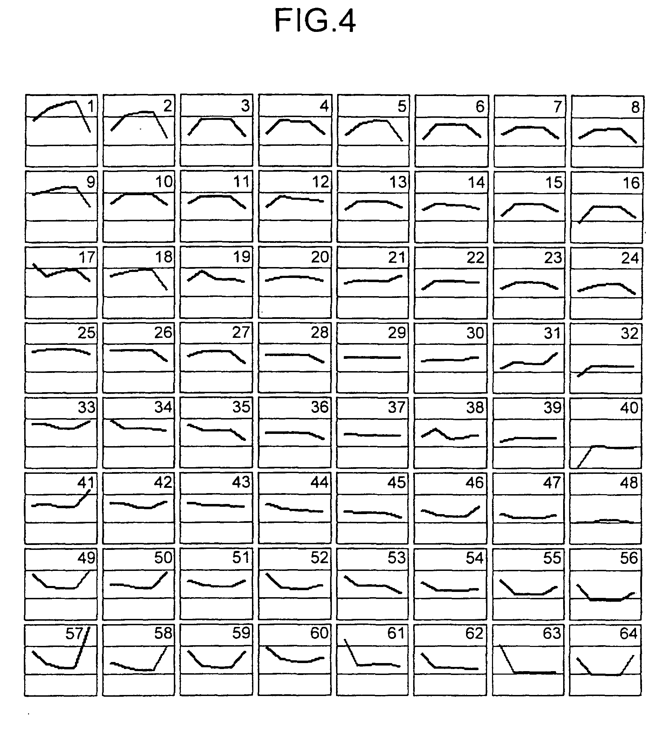

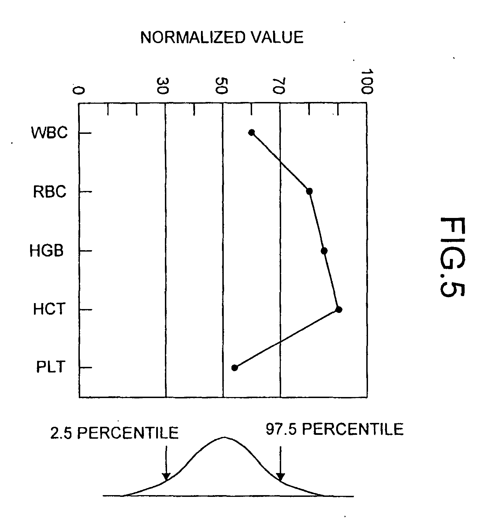

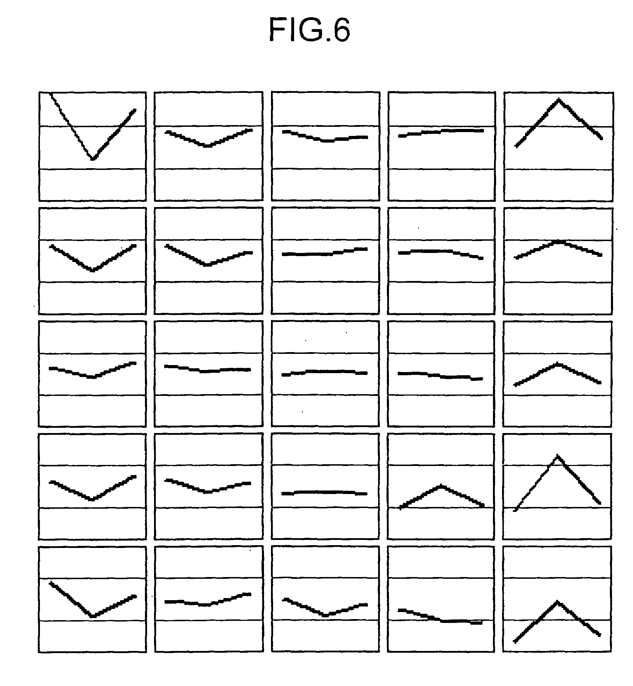

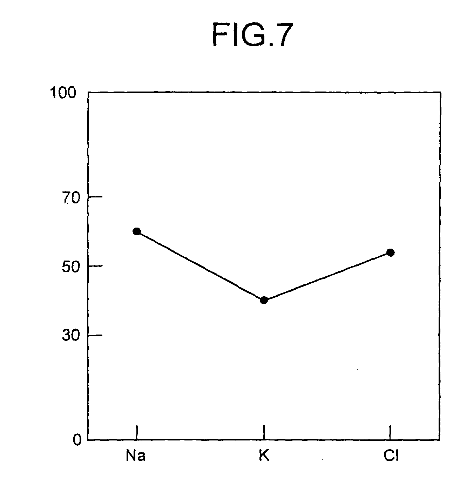

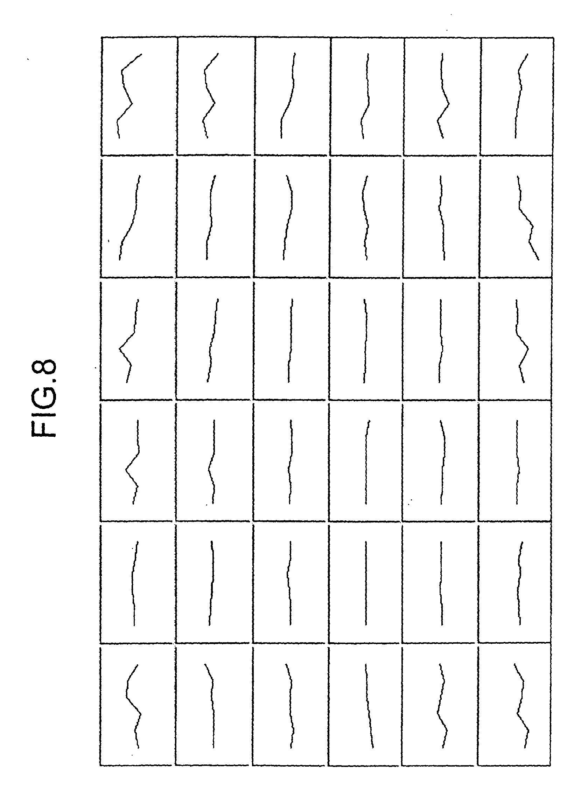

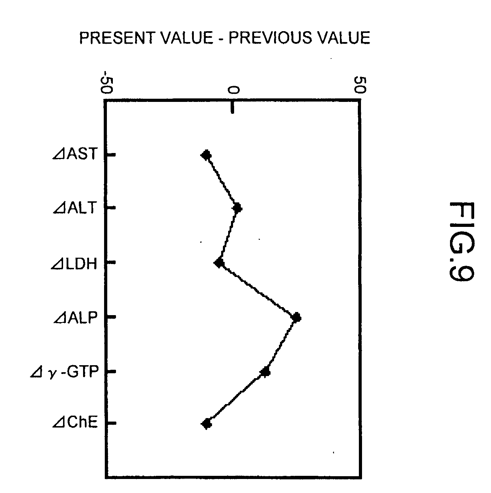

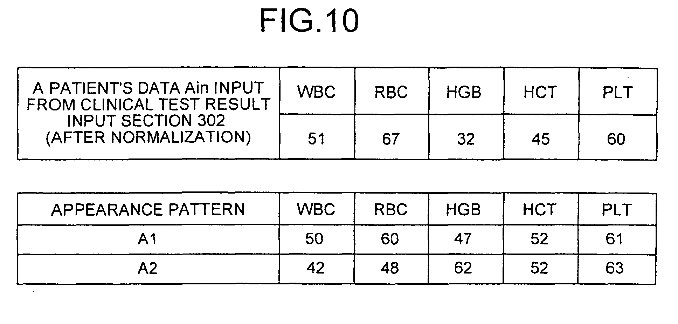

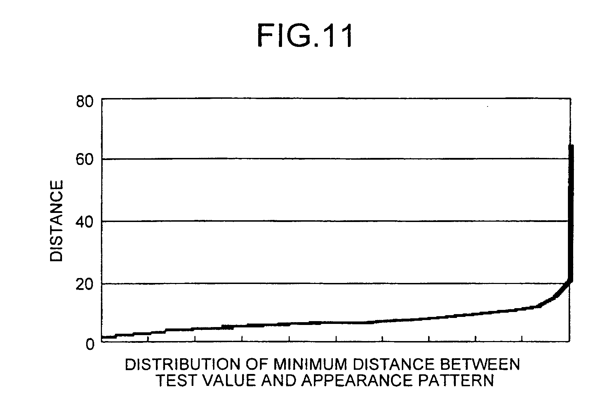

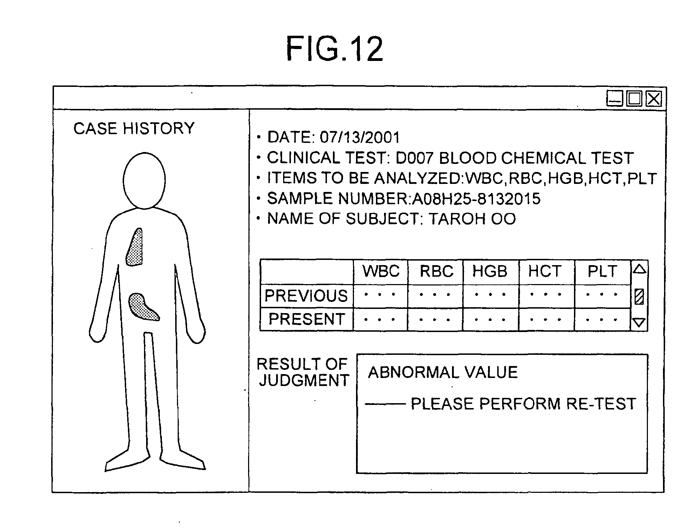

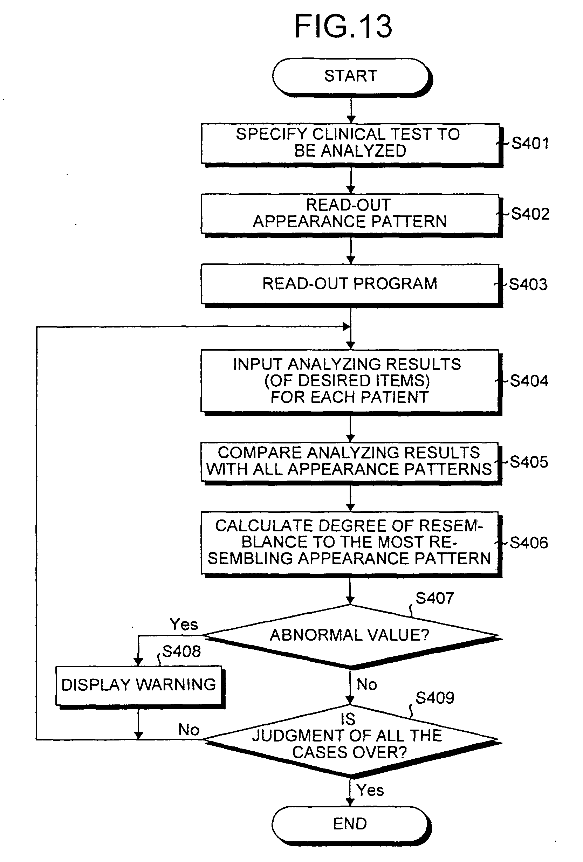

Fig. 1 illustrates an example of the exterior layout of a clinical test analyzing device, according to the present invention, connected to an automatic analyzing device; Fig. 2 is a schematic diagram representing an example of the hardware configuration of the clinical test analyzing device; Fig. 3 is a schematic diagram of an example of the functional configuration of the clinical test analyzing device; Fig. 4 illustrates 64 appearance patterns on analyzing results of 5 blood test items; Fig. 5 is a magnified diagram of one of the appearance patterns illustrated in Fig. 4; Fig. 6 illustrates appearance patterns of 25 items on analyzing results of 3 electrolyte test items; Fig. 7 is a magnified diagram of one of the appearance patterns illustrated in Fig. 6; Fig. 8 represents 36 appearance patterns on analyzing results of 6 blood chemical test items; Fig. 9 is a magnified diagram of one of the appearance patterns illustrated in Fig. 8; Fig. 10 is a table of analyzing results of 5 prescribed items in blood test and numerical values of appearance patterns to be compared; Fig. 11 represents distribution of the degree of resemblance (minimum distance) of data of the population (approx. 40,000 cases) used when extracting the appearance patterns illustrated in Fig. 4, calculated using the appearance patterns shown in the figure; Fig. 12 illustrates an example of the warning display screen; and Fig. 13 is a flowchart representing a process example of the clinical test analyzing device.

BEST MODE FOR CARRYING OUT THE INVENTION

Exemplary embodiments of the present invention are explained in detail below referring to the accompanying drawings.

An explanation is given here about the clinical test analyzing device, the clinical test analyzing method, and the computer program, according to the present invention, with an example where the clinical test analyzing device is connected to an automatic analyzing device in a hospital.

Fig.1 illustrates an example of the exterior layout of the clinical test analyzing device, according to the present invention, connected to an automatic analyzing device. The clinical test analyzing device 100 judges whether a patient needs a re-test or not, by inputting clinical test analyzing results of a plurality of patients, which are output from the automatic analyzing device 200 at any time. The automatic analyzing device 200 performs clinical tests for samples from patients and outputs the analyzing results to the clinical test analyzing device 100.

The clinical test here means mainly a sample test including a plurality of analyzing items for samples provided from a subject (since the present embodiment assumes an application example at a hospital, a subject means a patient). Classification of the sample test can be made, for example, by employing a classification according to the NHI point, such as D000: urine standard quantitative test, D0001: urine special quantitative test, D0002: microscopic examination of urinary sediment, D0003: fecal examination, D0004: punctured/collected fluid test, D0005: blood morphology/function test, D0006: hemorrhage/blood coagulation test, D0007: blood chemical test, D0008: endocrinological test, D0009: tumor marker, D0010: special analysis, D0011: immunity hematological test, D0012: infection serologic test, D0013: hepatitis virus related test, D0014: self-antibody test, D0015: immunological test for plasma protein, D0016: cell function test, D0017: microscopic examination of bacteria of excrement, effusion, or secretion, D0018: bacterial cultivation identification test, D0019: bacteria agent susceptibility test, D0020: acid-fast bacteria separation cultivation test, D0021: acid-fast bacteria identification test, D0022: acid-fast bacteria agent susceptibility test, D0023: microorganism nucleic acid identification/quantitative test, D0024: animal test, etc. Furthermore, a plurality of analyzing items is included in each clinical test. The analyzing items indicate a plurality of test items or analyzing items constituting a clinical test, for instance, a measurement of sodium (Na) concentration in an electrolyte.

The following describes the outline of the automatic analyzing device 200 and the process of obtaining analyzing results. The automatic analyzing device 200, as illustrated in Fig. 1, includes an analyzing section 201, a consumables storing section 202, an interpreting section 203, a display section 204, and a transmission line 205. The transmission line 205 is used for sending analyzing results to the clinical test analyzing device 100.

The analyzing section 201 performs a spectroscopic analysis and light absorption analysis by dripping a reagent on the sample in a reaction container, and outputs analyzing results by colorimetry and nephelometry. The analyzing section 201 also automatically conveys reaction containers, and if necessary, leads unused reaction containers to an analyzing path. Furthermore, the analyzing section 201 disposes of reaction containers to an assorted waste material storage upon completion of the test. The analyzing section 201 also cleans the interior of the device using cleaning probes and previously stored cleanser, so that the next test goes on without being interrupted.

The consumables storing section 202 stores various kinds of consumables used for clinical test or chemical analysis. The consumables include reagents for performing various kinds of analysis, cleansers, containers, and parts to be changed periodically.

The interpreting section 203, if necessary, classifies contents of the test items, based on analyzing results obtained from the analyzing section 201. For example, in the analyzing section 201, when dripping a reagent A (coloration medium) into a reagent B (enzyme labeling reagent), reagent A, reagent B, and antigen existing in the sample make a formation of immunity compound. Then a colored compound C is formed by the action of an enzyme that is combined to the reagent B to quantify the amount of antigen in the sample using colorimetry. Particularly, when a clinical test is a prescribed blood test, there are a number of test items, and the reagents may be overlapped in the middle of the test. The intermediate results, therefore, are sequentially stored, and the interpreting section 203 makes a lump-sum output of a plurality of analyzing results using a preset computational formula.

The display section 204 displays various kinds of information. The information displayed by the display section 204 includes the title of test, the test items, the date of test, the number of tests (the number of samples), the process status of test, etc. Besides, if any error or interruption occurs during the test, the display section 204 displays the content of error, the status of device, and the procedure of corrective actions to recover.

The analyzing results of the clinical test that are output from the analyzing section 201 or the interpreting section 203 are sent to the clinical test analyzing device 100 via the transmission line 205. The transmission line 205 may be a local-area-network (LAN), a telephone line, or a universal serial bus (USB), etc. The sending scheme is not limited to a specific method, as long as analyzing results are sent promptly to the clinical test analyzing device 100. The present embodiment utilizes a LAN connection for transmitting data.

The automatic analyzing device 200 outputs the analyzing results of a plurality of items for a plurality of patients. Since the automatic analyzing device 200 is installed in a hospital, the population of interest is a group of people who have certain kind of disease, and in most cases, many items show numerical values deviated from those of a healthy person. Therefore, it is meaningless to perform a re-test simply because a subject has an abnormal value; rather, it is necessary to understand the tendency of abnormal values to judge whether the abnormal value requires a re-test.

Furthermore, the patients have various kinds of diseases, such as hepatic disease, pulmonary disease, etc. Depending on the type of disease, items with abnormal values may show a tendency, in other words, a correlation may appear on a plurality of items. The clinical test analyzing device 100 performs a judgment whether the abnormal value definitely requires a re-test or a further precise test, by referring to a database where such correlations are phenomenologically determined.

Fig. 2 is a schematic diagram representing an example of the hardware configuration of the clinical test analyzing device 100. The clinical test analyzing device 100 includes a central processing unit (CPU) 101, a random access memory (RAM) 102, a read only memory (ROM) 103, a hard disc (HD) 104, a network interface card (NIC) 105, a video card 106, and a peripheral interface (I/F) section 107. A mouse 171 and a key board 172 are connected to the peripheral interface section 107. A cathode ray tube (CRT) (see Fig. 1) is connected to the video card 106.

The HD 104 includes a program section 141 and a database section 142. The program section 141 stores an operating system (OS) to basically control the entirety of the clinical test analyzing device 100, a communication program, a resemblance calculation program, a normalization conversion program, a judgment program, and a warning display program, etc. The database section 142 stores appearance patterns according to the type of clinical test (for example, appearance pattern database (hereinafter, "DB") for D0005 blood morphology/function test, appearance pattern DB for D0007 blood chemical test, etc.) and judgment thresholds corresponding to the appearance patterns (for example, judgment threshold for D0005 blood morphology/function test, judgment threshold for D0007 blood chemical test, etc.).

The following explains the functional configuration of the clinical test analyzing device 100 and the concrete relationship of each functional section with the hardware illustrated in Fig. 2. Fig. 3 is a schematic diagram of an example of the functional configuration of the clinical test analyzing device 100. The functional configuration of the clinical test analyzing device 100 includes an appearance pattern storing section 301, a clinical test result input section 302, a resemblance calculation section 303, and a re-test judgment section 304.

The appearance pattern storing section 301 stores a plurality of appearance patterns on analyzing results of a prescribed plurality of items in clinical test for a prescribed population. The prescribed population in the present embodiment means inpatients. Besides, since many types of appearance patterns are stored, it is not necessary to prepare for appearance patterns obtained from a population of patients with a specific disease among the inpatients. Namely, the appearance pattern storing section 301 stores all types of appearance patterns, such as appearance patterns shown on a patient with hepatic disease or pulmonary disease, etc. In other words, an operator of the clinical test analyzing device 100 does not need to be aware of the set of appearance patterns to be used for a test. Depending on the type of usage, however, the operator can select appropriate appearance patterns based on the patient to be judged, to speed up the judgment of the re-test judgment section 304.

Since the appearance patterns are those of analyzing results of "a prescribed plurality of items" in a clinical test, it is necessary to use different appearance patterns when analyzing different items. Namely, when the analyzing items are a, b, c, then appearance patterns of A1, A2, ..., An (hereinafter, "group A") can be used regardless of the patient. However, when the analyzing items are $\alpha$, $\beta$, $\gamma$, then appearance patterns of group B (B1, B2, ..., Bm) should be used, which are different from the appearance patterns of group A.

Fig. 4 is an illustration of 64 appearance patterns on analyzing results of 5 blood test items. Fig. 5 is a magnified diagram of one of the appearance patterns illustrated in Fig. 4. On the other hand, Fig. 6 is an illustration of appearance patterns of 25 items on analyzing results of 3 electrolyte test items. Fig. 7 is a magnified diagram of one of the appearance patterns illustrated in Fig. 6.

In.the graph illustrated in Fig. 5, 5 analyzing items in D0005 blood morphology/function test are plotted on the horizontal axis. These analyzing items include white blood corpuscle (WBC), red blood corpuscle (RBC), hemoglobin (HGB), hematocrit (HCT), and blood platelet (PLT). Values of each analyzing item that are normalized by appearance probability are plotted on the vertical axis. In the graph illustrated in Fig. 7, 3 analyzing items in D0007 blood chemical test are plotted on the horizontal axis. These analyzing items include sodium (Na), potassium (K), and chloride (Cl). Values of each analyzing item that are normalized by appearance probability are plotted on the vertical axis. For the normalization, the values 30 and 70 on the vertical axis are set to be 2.5 percentile and 97.5 percentile, respectively.

When the automatic analyzing device 200 outputs analyzing results of blood test (blood morphology/function test) including the 5 items illustrated in Fig. 5, the appearance pattern group of blood test illustrated in Fig. 4 can be used for analysis. On the other hand, when the automatic analyzing device 200 outputs analyzing results of electrolyte test (blood chemical test) including the 3 items illustrated in Fig. 7, the appearance pattern group of the electrolyte test illustrated in Fig. 6 can be used.

The appearance patterns illustrated in Fig. 4 were obtained using the self organization map (SOM) with blood test data of patients in 40,000 cases. The self organization map is known as an efficient method to visualize useful information implied in nonlinear multivariate data, and is a helpful map for capturing correlation of each item when analyzing results of mufti-items (nonlinear multivariate) are output, just like the case of the automatic analyzing device 200. SOM is a kind of the so called data mining. Therefore, if it is possible to obtain appearance patterns that can be used to compute the degree of resemblance using data mining, any method other than SOM can be used. The data mining technique has made it possible to judge correlation of numerical values, which, in the past, was not simple to do without being a skilled doctor.

Further, in terms of the number of appearance patterns, Fig. 4 and Fig. 6 display 64 patterns and 25 patterns, respectively. These numbers, however, are arbitrary and can be properly determined according to the number of patients or the number of items (5 items in the blood test).

In the above example, appearance patterns were obtained from the analyzing results of a plurality of items in one-time test of each patient ("the present value result"). However, the appearance pattern, not limited to the above case, can be constructed by taking the patient's history into consideration. For example, inpatients may have to take a periodic blood test. Even though an item here shows an abnormal value compared with the last test result, if another item also shows an abnormal value within certain range, it may be judged that the appearance pattern is acceptable. Therefore, even when the present value result leads to a judgment that a re-test is definitely needed, performing a comparison between the present test results and the previous test results leads to a judgment that the appearance patterns can be accepted, making it possible to eliminate an unnecessary re-test.

Fig. 8 is a representation of 36 appearance patterns on analyzing results of 6 blood chemical test items. Fig. 9 is a magnified diagram of one of the appearance patterns illustrated in Fig. 8.

In the graph illustrated in Fig. 9, 6 analyzing items in D0007 blood chemical test are plotted on the horizontal axis. These analyzing items include asparate amino-transferase (AST), alanine amino-transferase (ALT), lactic dehydrogenase (LDH), alkaline phosphatase (ALP), γ-glutamyltranspeptidase (γ-GTP), cholinesterase (ChE). Differences between the present value and the previous value are plotted on the vertical axis. The ratio of the present value to the previous value can also be used to construct appearance patterns depending on the type of usage, although the difference between the present value and the previous value is used in this example. It is worth to take the patient's history into consideration particularly when, for example, changing the medicine to be applied in certain stage of pharmaceutical therapy.

Referring to Fig. 2 and 3, the appearance pattern storing section 301 can be implemented using, for example, the database section 142 of the HD 104. Furthermore, the database section 142 stores a number of appearance patterns corresponding to each classification according to the NHI point.

The clinical test result input section 302 that inputs analyzing results of a plurality of items for each patient can be implemented using, for example, the NIC 105, the CPU 101, and the communication program stored in the program section 141 of the HD 104.

The resemblance calculation section 303 calculates the degree of resemblance of analyzing results to the most resembling appearance pattern, by comparing the analyzing results from the clinical test result input section 302 and a plurality of appearance patterns stored in the appearance pattern storing section 301. When the appearance patterns illustrated in Fig. 4 are used, where 5 items are digitized, a total of distances between the five numerical values input from the clinical test result input section 302 and the numerical values of items corresponding to the comparing appearance patterns are obtained for all of the appearance patterns, and then the minimum value is determined as the degree of resemblance.

Fig. 10 is a table of analyzing results of 5 prescribed items in blood test and numerical values of appearance patterns to be compared. Now, let us assume that there are only two appearance patterns, to make explanation simple. The degree of resemblance K1 between the analyzing result Ain and the appearance pattern A1 is given by,

$$K1=sqrt((51-50)2+(67-650)2+(32-47)2+(45-52)2+(60-61)2)\cong18.0$$

3.

On the other hand, the degree of resemblance K2 between the analyzing result Ain and the appearance pattern A2 is given by,

$$K2=sqrt((51-42)2+(67-48)2+(32-62)2+(45-52)2+(60-63)2)\cong37.54.$$

Therefore, in this example, the degree of resemblance of the analyzing result Ain is taken as the smaller value, 18.03.

Conventionally, even if one item is out of the preset range, it is announced that the patient needs to take a re-test or a further precise test. In other words, the conventional method incorporates an independent judgment algorithm without taking into consideration the correlation between items. The present method, however, provides a comprehensive judgment algorithm by taking into consideration the correlation between items.

Although the degree of resemblance is obtained based on the total of distances between items in the above explanation, the method of calculation is not limited to the method using the total of distances. For example, the degree of resemblance can also be calculated based on any 2 items that have the largest difference in distance, or even based on the size or the volume of area. Namely, in the resemblance calculation section 303, the calculation method is not limited to a specific algorithm, as long as the degree of resemblance to the most resembling appearance pattern can be calculated among a plurality of comparing appearance patterns.

The following is an explanation about the appearance patterns illustrated in Fig. 4 and the degree of resemblance. Fig. 11 is a graph representing distribution of the degree of resemblance (minimum distance) of data from the population (approx. 40,000 cases) used when extracting the appearance patterns of Fig. 4, calculated using the appearance patterns illustrated in the figure. The serial numbers of data are plotted on the horizontal axis, and the corresponding minimum distances are plotted on the vertical axis. The data on the horizontal axis are sorted in ascending order of distance. As shown in the figure, since most patients have a small value of the minimum distance, they do not need a re-test or a further precise test. On the other hand, the right edge of the graph shows an abrupt rising of the minimum distance, and it can be considered that a re-test or a further precise test should be performed for the patient having the data.

The resemblance calculation section 303 can be implemented using, for example, the CPU 101 and the resemblance calculation program stored in the program section 141 of the HD 104. In the examples of Fig. 4 and Fig. 6, appearance patterns are normalized, and therefore, when calculating the degree of resemblance, Ain also needs to be converted to the same scale to be compared. The conversion formula is also stored in the program section 141 of the HD 104 as a normalization conversion program.

The re-test judgment section 304 judges whether the clinical test result of interest needs a re-test or a further precise test, based on the degree of resemblance calculated by the resemblance calculation section 303 and a preset judgment threshold. As illustrated in Fig. 11, the data from patients, in most cases, are either virtually identical or similar to the appearance patterns. However, there rarely are measurement values that do not match with any appearance pattern.

In the example of Fig. 11, if the judgment threshold is set to 20, it is possible to efficiently detect samples that need a re-test or a further precise test.

When it is judged that a re-test is needed, a warning is displayed on the CRT of the clinical test analyzing device 100 (see Fig. 1). Fig. 12 is an illustration of an example of the warning display screen. The screen configuration of the warning display program contributes to the judgment of a doctor or a test technician by displaying an indication whether the result is an abnormal value, and other data such as the case history, the date, the class of clinical test, items to be analyzed, the sample number, the name of subject, the present value, and the previous value.

Generally, the most frequent cases of outputting an abnormal value are when subjects or samples to be tested are mixed up. Therefore, if the clinical test analyzing device 100 according to the present invention is used, it is also possible to prevent such an absurd medical mistake. Furthermore, since the automatic analyzing device 200 automatically conducts and completes various kinds of analyzing processes in a short time, even in the case that the subjects were mixed up, it is possible for the subjects to take a re-test during their stay in the hospital. Therefore there is less burden on the subject.

The re-test judgment section 304 can be implemented using, for example, the CPU 101, the judgment program and warning display program stored in the program section 141 of the HD 104, and the judgment threshold stored in the database section 142.

Lastly, an operation example of the clinical test analyzing device 100 is explained with a flow chart. Fig. 13 is the flow chart representing a process example of the clinical test analyzing device. The operator, for example a test technician, specifies the clinical test to be analyzed (step S401). For example, D0001: urine standard quantitative test can be specified. Following the instruction, the corresponding appearance patterns and the judgment threshold are read from the database section 142 and stored in the RAM 102 (step S402).

Subsequently, the communication program, the resemblance calculation program, the normalization conversion program, and the judgment program are read from the program section 141, and are stored in the RAM 102 and the cache of the CPU 101 (step S403). Now that the preparation for conducting the test is done, the CPU 101 sequentially inputs the analyzing results from the automatic analyzing device 200 for every single subject (step S404).

The analyzing results input for every patient are converted by the normalization conversion program, and then compared with all of the appearance patterns (step S405). The resemblance calculation program calculates the degree of resemblance to the most resembling appearance pattern in the comparison process (step S406). When the degree of resemblance exceeds the judgment threshold (step S407), the warning display program is called from the program section 141 to display a warning, as illustrated in Fig. 12 (step S408). Namely, by executing the steps S405 through S 408, a judgment is made based on the plurality of appearance patterns and the analyzing results for every single patient, whether a re-test or a further precise test is needed, by detecting the analyzing result with a slim chance of occurrence.

Finally when the judgment of all the cases is over (step S409), the process is terminated.

As described above, since the clinical test analyzing device 100 calculates the degree of resemblance based on most likely patterns, it is possible to easily make a comprehensive judgment whether a re-test or a further precise test is definitely necessary. Although a population of patients is used in the above example, a population of healthy people can also be used, without being limited to any specific population. Examples of this kind of application can be found in clinical tests for different areas or different age groups.

The clinical test analyzing device 100 inputs a considerable amount of clinical test data from the automatic analyzing device 200. These data can be stored in the HD 142, and if necessary, can be used to reconstruct the appearance patterns using the SOM. The appearance patterns can be constructed using any appropriate software.

As described above, since the appearance pattern storing section stores a plurality of appearance patterns on analyzing results of a prescribed plurality of items in a clinical test for a prescribed population, the clinical test result input section inputs the analyzing results of the prescribed plurality of items in a clinical test for a subject, and the resemblance calculation section calculates the degree of resemblance of the analyzing result to the most resembling appearance pattern, based on the analyzing result that is input by the clinical test result input section and a plurality of appearance patterns stored in the appearance pattern storing section, it is possible to judge whether a re-test is definitely necessary or not, based on the appearance patterns, and thereby it is possible to rationally eliminate unnecessary re-tests while maintaining reliability of the test.

Since the clinical test analyzing device according to the present invention takes a population of patients as the prescribed population, it is possible to judge whether an abnormal value is an acceptable pattern or a test result that requires a definite re-test, based on the abnormal value pattern. Thereby it is possible to rationally eliminate unnecessary re-tests while maintaining reliability of the test.

Since the plurality of appearance patterns of the clinical test analyzing device according to the present invention is extracted from the prescribed population, it is possible to obtain a pattern of phenomenologically strong correlation on analyzing results of the plurality of items, and judge whether it is an acceptable pattern or a test result that requires a definite re-test. Thereby, it is possible to rationally eliminate unnecessary re-tests while maintaining reliability of the test.

Since the clinical test for the clinical test analyzing device according to the present invention is either at least one of tests or a combination of more than one test among microscopic examination of urinary sediment, blood morphology/function test, hemorrhage/blood coagulation test, blood chemical test, endocrinological test, tumor marker, infection serologic test, self-antibody test, and immunological test for plasma protein, it is possible to judge whether an abnormal value is an acceptable pattern or a test result that requires a definite re-test, based on the appearance patterns corresponding to the classification of clinical tests according to the health insurance. Thereby it is possible to rationally eliminate unnecessary re-tests while maintaining reliability of the test.

Since the prescribed plurality of items for the clinical test analyzing device according to the present invention are values of numerical quantity or concentration of white blood corpuscle, red blood corpuscle, hemoglobin, hematocrit, and blood platelet, it is possible to judge whether an abnormal value is an acceptable pattern or a test result that requires a definite re-test, using the appearance patterns based on the blood test that is most frequently conducted in the medical field in practice. Thereby it is possible to rationally eliminate unnecessary re-tests while maintaining reliability of the test.

Since the prescribed plurality of items for the clinical test analyzing device according to the present invention are values of concentration of sodium (Na), potassium (K), and chloride (Cl) in an electrolyte, it is possible to judge whether an abnormal value is an acceptable pattern or a test result that requires a definite re-test, using the appearance patterns based on the electrolyte test that is most frequently conducted in the medical field in practice. Thereby it is possible to rationally eliminate unnecessary re-tests while maintaining reliability of the test.

Since the analyzing results of the prescribed plurality of items for the clinical test analyzing device according to the present invention are the analyzing results that are compared with those of the previous test in item by item for every single member of the prescribed population, it is possible to judge whether an abnormal value is an acceptable pattern or a test result that requires a definite re-test, based on the appearance patterns that take into consideration, the previous clinical test results and time variation of the test value. Thereby it is possible to rationally eliminate unnecessary re-tests while maintaining reliability of the test.

Since the re-test judgment section of the clinical test analyzing device according to the present invention makes a judgment whether the subject needs a re-test, based on the degree of resemblance calculated by the resemblance calculation section and the judgment threshold, it is possible to judge whether an abnormal value is an acceptable pattern or a test result that requires a definite re-test, when the test result shows that the degree of resemblance to any appearance pattern is small (i.e., possibility of being abnormal is quite high). Thereby it is possible to rationally eliminate unnecessary re-tests while maintaining reliability of the test.

Since the clinical test analyzing method according to the present invention inputs analyzing results of a prescribed items in a clinical test for a subject at the clinical test result inputting step, reads out a plurality of appearance patterns on analyzing results of the prescribed plurality of items in a clinical test for a prescribed population at the appearance pattern reading step, and detects the analyzing result in question is an abnormal result of a slim chance of occurrence at the abnormal result detecting step, based on the plurality of appearance patterns read at the step of reading and the analyzing result input at the step of inputting, it is possible to judge whether a re-test is definitely necessary, based on the appearance patterns. Thereby it is possible to rationally eliminate unnecessary re-tests while maintaining reliability of the test.

Since the clinical test analyzing method according to the present invention takes a population of patients as the prescribed population, it is possible to judge whether an abnormal value is an acceptable pattern or a test result that requires a definite re-test, based on the abnormal value pattern, and thereby it is possible to rationally eliminate unnecessary re-tests while maintaining reliability of the test.

Since the plurality of appearance patterns of the clinical test analyzing method according to the present invention are extracted from the prescribed population, it is possible to obtain a pattern of phenomenologically strong correlation on analyzing results of the plurality of items, and judge whether it is an acceptable pattern or a test result that requires a definite re-test. Furthermore, when calculating appearance patterns, it is possible to use the data mining method, such as the machine learning, the neural network, and the self organization map, and thereby it is possible to rationally eliminate unnecessary re-tests while maintaining reliability of the test.

Since the analyzing results of the prescribed plurality of items for the clinical test analyzing method according to the present invention are the analyzing results that are compared with those of the previous test in item by item for every single member of the prescribed population, it is it possible to judge whether an abnormal value is an acceptable pattern or a test result that requires a definite re-test, based on the appearance patterns that takes the previous clinical test results and time variation of the test value into consideration, and thereby it is possible to rationally eliminate unnecessary re-tests while maintaining reliability of the test.

Since the clinical test analyzing program according to the present invention makes a computer realize the steps of inputting analyzing results of a prescribed items in a clinical test for a subject, reading a plurality of appearance patterns on analyzing results of the prescribed plurality of items in a clinical test for a prescribed population, and detecting whether the analyzing result in question is an abnormal result of a slim chance of occurrence or not, based on the plurality of appearance patterns read at the step of reading and the analyzing result input at the step of inputting, it is possible to judge whether a re-test is definitely necessary or not, based on the appearance patterns, and thereby it is possible to rationally eliminate unnecessary re-tests while maintaining reliability of the test.

Since the clinical test analyzing program according to the present invention takes a population of patients as the prescribed population, it is possible to judge whether an abnormal value is an acceptable pattern or a test result that requires a definite re-test, based on the abnormal value pattern, and thereby it is possible to rationally eliminate unnecessary re-tests while maintaining reliability of the test.

Since the plurality of appearance patterns of the clinical test analyzing program according to the present invention is extracted from the prescribed population, it is possible to obtain a pattern of phenomenologically strong correlation on analyzing results of the plurality of items, and judge whether it is an acceptable pattern or a test result that requires a definite re-test, and thereby it is possible to rationally eliminate unnecessary re-tests while maintaining reliability of the test.

Since the analyzing results of the prescribed plurality of items for the clinical test analyzing method according to the present invention are the analyzing results that are compared with those of the previous test in item by item for every single member of the prescribed population, it is possible to judge whether an abnormal value is an acceptable pattern or a test result that requires a definite re-test, based on the appearance patterns that takes the previous clinical test results and time variation of the test value into consideration, and thereby it is possible to rationally eliminate unnecessary re-tests while maintaining reliability of the test.

INDUSTRIAL APPLICABILITY

As described above, the clinical test analyzing device, the clinical test analyzing method, and the computer program according to the present invention are suitable for analyzing a plurality of analyzing items with a plurality of appearance patterns.

**Claims**

1. A clinical test analyzing device comprising:

   an appearance pattern storing section that stores a plurality of appearance patterns on past analyzing results of a plurality of prescribed items in a clinical test for a prescribed population;
   a clinical test result input section that acquires current analyzing results of the plurality of prescribed items in the clinical test for a subject; and
   a resemblance calculation section that calculates the degree of resemblance of the current analyzing results to a most resembling appearance pattern, by comparing the current analyzing results with the appearance patterns.

2. The clinical test analyzing device according to claim 1, wherein the prescribed population is a population of subjects having illness.

3. The clinical test analyzing device according to claim 1 or 2, wherein the appearance patterns are extracted from the prescribed population.

4. The clinical test analyzing device according to claim 1 or 2, wherein the clinical test is one or more selected from a group comprising of microscopic examination of urinary sediment, blood morphology/function test, hemorrhage/ blood coagulation test, blood chemical test, endocrinological test, tumor marker, infection serologic test, self-antibody test, and immunological test for plasma protein.

5.  The clinical test analyzing device according to claim 1 or 2, wherein the prescribed items are values of numerical quantity or concentration of white blood corpuscle, red blood corpuscle, hemoglobin, hematocrit, and blood platelet.

6.  The clinical test analyzing device according to claim 1 or 2, wherein the prescribed items are values of concentration of sodium (Na), potassium (K), and chloride (Cl) in an electrolyte.

7.  The clinical test analyzing device according to claim 1 or 2, wherein the current analyzing results are compared item by item with the analyzing results of the previous test for every member of the prescribed population.

8.  The clinical test analyzing device according to claim 1 or 2, further comprising a re-test judgment section that judges whether the subject needs a re-test, based on the degree of resemblance and a judgment threshold.

9.  A clinical test analyzing method comprising:

    acquiring current analyzing results of a plurality of prescribed items in a clinical test for a subject;
    acquiring a plurality of appearance patterns on past analyzing results of the prescribed items in the clinical test for a prescribed population; and
    detecting whether each of the current analyzing results is an abnormal result of a slim chance of occurrence by comparing the current analyzing results with the appearance patterns.

10. The clinical test analyzing method according to claim 9, wherein the prescribed population is a population of subjects having illness.

11. The clinical test analyzing method according to claim 9 or 10, further comprising extracting the appearance patterns from the prescribed population.

12. The clinical test analyzing method according to claim 9 or 10, further comprising comparing the current analyzing results item by item with the analyzing results of the previous test for every member of the prescribed population.

13. A computer program making a computer realize:

    acquiring current analyzing results of a plurality of prescribed items in a clinical test for a subject;
    acquiring a plurality of appearance patterns on past analyzing results of the prescribed items in the clinical test for a prescribed population; and
    detecting whether each of the current analyzing results is an abnormal result of a slim chance of occurrence by comparing the current analyzing results with the appearance patterns.

14. The computer program according to claim 13, wherein the prescribed population is a population of subjects having illness.

15. The computer program according to claim 13 or 14, further making the computer realize extracting the appearance patterns from the prescribed population.

16. The computer program according to claim 13 or 14, further making the computer realize comparing the current analyzing results item by item with the analyzing results of the previous test for every member of the prescribed population.

FIG.1

# FIG.2

100 · FROM AUTOMATIC ANALYZING DEVICE 200

| 101 | 102 | 103 | 105 | 171 | 172 |
|---|---|---|---|---|---|
| | | | | MOUSE | KEYBOARD |
| CPU | RAM | ROM | NIC | PERIPHERAL I/F | ~107 |

104

106

VIDEO CARD

TO CRT

**HD**

141

142

| PROGRAM SECTION | DATABASE SECTION |
|---|---|
| **OS** | ⋮ |
| COMMUNICATION PROGRAM | D005 APPEARANCE PATTERN DB FOR BLOOD MORPHOLOGY/ FUNCTION TEST |
| RESEMBLANCE CALCU-LATION PROGRAM | ⋮ |
| NORMALIZATION CONVERSION PROGRAM | D007 APPEARANCE PATTERN DB FOR BLOOD CHEMICAL TEST |
| JUDGMENT PROGRAM | ⋮ |
| WARNING DISPLAY PROGRAM | D005 JUDGMENT THRESHOLD FOR BLOOD MORPHOLOGY /FUNCTION TEST |
| ⋮ | ⋮ |
| | D007 JUDGMENT THRESHOLD FOR BLOOD CHEMICAL TEST |
| | ⋮ |

EP 1 413 888 A1

# FIG.3

100

FROM AUTOMATIC
ANALYZING DEVICE 200

301

| APPEARANCE PATTERN STOR- ING SECTION |

302

| CLINICAL TEST RESULT INPUT SECTION |

A PLURALITY OF
APPEARANCE
PATTERNS

ANALYZING
RESULTS OF A
PLURALITY OF
ITEMS

| RESEMBLANCE CALCULATION SECTION | ~303

DEGREE OF RESEMBLANCE

| RE-TEST JUDGMENT SECTION | ~304

RESULTS OF JUDGMENT

# FIG.4

# FIG.5

# FIG.6

# FIG.7

FIG.8

# FIG.9

# FIG.10

| A PATIENT'S DATA Ain INPUT FROM CLINICAL TEST RESULT INPUT SECTION 302 (AFTER NORMALIZATION) | WBC | RBC | HGB | HCT | PLT |
|---|---|---|---|---|---|
| | 51 | 67 | 32 | 45 | 60 |

| APPEARANCE PATTERN | WBC | RBC | HGB | HCT | PLT |
|---|---|---|---|---|---|
| A1 | 50 | 60 | 47 | 52 | 61 |
| A2 | 42 | 48 | 62 | 52 | 63 |

# FIG.11

DISTRIBUTION OF MINIMUM DISTANCE BETWEEN
TEST VALUE AND APPEARANCE PATTERN

# FIG.12

CASE HISTORY

- DATE: 07/13/2001
- CLINICAL TEST: D007 BLOOD CHEMICAL TEST
- ITEMS TO BE ANALYZED:WBC,RBC,HGB,HCT,PLT
- SAMPLE NUMBER:A08H25-8132015
- NAME OF SUBJECT: TAROH OO

|  | WBC | RBC | HGB | HCT | PLT |
|---|---|---|---|---|---|
| PREVIOUS | · · · | · · · | · · · | · · · | · · · |
| PRESENT | · · · | · · · | · · · | · · · | · · · |

RESULT OF JUDGMENT

ABNORMAL VALUE

——— PLEASE PERFORM RE-TEST

# FIG.13

```
                    ┌──────────────┐
                    │    START     │
                    └──────┬───────┘
                           │
                           ▼
          ┌────────────────────────────────┐
          │ SPECIFY CLINICAL TEST TO       │─── S401
          │ BE ANALYZED                    │
          └────────────────┬───────────────┘
                           │
                           ▼
          ┌────────────────────────────────┐
          │ READ-OUT                       │─── S402
          │ APPEARANCE PATTERN             │
          └────────────────┬───────────────┘
                           │
                           ▼
          ┌────────────────────────────────┐
          │ READ-OUT PROGRAM               │─── S403
          └────────────────┬───────────────┘
                           │
                           ▼
          ┌────────────────────────────────┐
          │ INPUT ANALYZING RESULTS        │─── S404
          │ (OF DESIRED ITEMS)             │
          │ FOR EACH PATIENT               │
          └────────────────┬───────────────┘
                           │
                           ▼
          ┌────────────────────────────────┐
          │ COMPARE ANALYZING RESULTS      │─── S405
          │ WITH ALL APPEARANCE PATTERNS   │
          └────────────────┬───────────────┘
                           │
                           ▼
          ┌────────────────────────────────┐
          │ CALCULATE DEGREE OF RESEM-     │─── S406
          │ BLANCE TO THE MOST RE-         │
          │ SEMBLING APPEARANCE PATTERN    │
          └────────────────┬───────────────┘
                           │      S407
                           ▼
      Yes           ◇─────────────────◇
  ┌───────────────  │  ABNORMAL VALUE? │
  │   S408          ◇─────────────────◇
  ▼                          │ No
┌──────────────────┐         │      S409
│ DISPLAY WARNING  │         ▼
└────────┬─────────┘  ◇──────────────────◇
         │       No   │       IS          │
         └──────────  │ JUDGMENT OF ALL THE│
                      │    CASES OVER?     │
                      ◇──────────────────◇
                               │ Yes
                               ▼
                       ┌──────────────┐
                       │     END      │
                       └──────────────┘
```

| INTERNATIONAL SEARCH REPORT | International application No. |
|---|---|
| | PCT/JP02/07758 |

**A. CLASSIFICATION OF SUBJECT MATTER**
Int.Cl$^7$ G01N35/00, G01N33/48

*According to International Patent Classification (IPC) or to both national classification and IPC*

**B. FIELDS SEARCHED**

Minimum documentation searched (classification system followed by classification symbols)
Int.Cl$^7$ G01N35/00, G01N33/48

Documentation searched other than minimum documentation to the extent that such documents are included in the fields searched

| | | | |
|---|---|---|---|
| Jitsuyo Shinan Koho | 1922–1996 | Toroku Jitsuyo Shinan Koho | 1994–2002 |
| Kokai Jitsuyo Shinan Koho | 1971–2002 | Jitsuyo Shinan Toroku Koho | 1996–2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| A | WO 99/05503 A (Arch Dev Corp.), 04 February, 1999 (04.02.99), Full description & AU 8579498 A & EP 993269 A & US 6058322 A & JP 2001-511372 A | 1–16 |
| A | JP 06-096138 A (Olympus Optical Co., Ltd.), 08 April, 1994 (08.04.94), Full description & IT 1270978 B & DE 4331018 A | 1–16 |

☐ Further documents are listed in the continuation of Box C.     ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 18 October, 2002 (18.10.02) | 05 November, 2002 (05.11.02) |

| Name and mailing address of the ISA/ | Authorized officer |
|---|---|
| Japanese Patent Office | |
| Facsimile No. | Telephone No. |

Form PCT/ISA/210 (second sheet) (July 1998)